# EUROPÄISCHE PATENTSCHRIFT

(11) **EP 1 827 413 B1**
(45) Veröffentlichungstag und Bekanntmachung des Hinweises auf die Patenterteilung: **15.06.2011**
(21) Anmeldenummer: 05769996.9
(22) Anmeldetag: 08.08.2005
(51) Int. Cl.: A61K 31/20, A61K 31/385, A61K 47/48, A23L 1/30, A61P 19/02, A61P 29/00, A61P 3/10

(54) **VERWENDUNG VON LIPONSÄUREHALTIGEN CYCLODEXTRINKOMPLEXEN**
USE OF CYCLODEXTRIN COMPLEXES CONTAINING LIPOIC ACID
UTILISATION DE COMPLEXES DE CYCLODEXTRINES CONTENANT DE L'ACIDE LIPOIQUE

(30) Priorität: 17.12.2004 DE 102004060914
(43) Veröffentlichungstag der Anmeldung: 05.09.2007
(73) Patentinhaber: AlzChem Trostberg GmbH, 83308 Trostberg (DE)
(72) Erfinder: SCHÜTT, Wolfgang, 46483 Raesfeld (DE); PURPURA, Martin, 53227 Bonn (DE)
(74) Vertreter: Weiss, Wolfgang
(86) Internationale Anmeldenummer: PCT/EP2005/008577
(87) Internationale Veröffentlichungsnummer: WO 2006/066637

(56) Entgegenhaltungen:
- EP-A- 0 654 484
- WO-A-02/052955
- DE-A1- 4 035 442
- TRENTIN M ET AL: "Capillary zone electrophoresis study of cyclodextrin: Lipoic acid host-guest interaction" ELECTROPHORESIS, WEINHEIM, DE, Bd. 23, Nr. 24, Dezember 2002 (2002-12), Seiten 4117-4122, XP002325022 ISSN: 0173-0835
- JUNQUERA ELENA ET AL: "Thermodynamic analysis of the binding of a hepatoprotectant drug, thioctic acid, by beta-cyclodextrin" JOURNAL OF PHARMACEUTICAL SCIENCES, Bd. 88, Nr. 6, Juni 1999 (1999-06), Seiten 626-631, XP008054852 ISSN: 0022-3549
- TONG LIN-HIU ET AL: "INCLUSION COMPLEXES OF ALPHA AND BETA CYCLODEXTRIN WITH ALPHA LIPOIC ACID" JOURNAL OF INCLUSION PHENOMENA AND MOLECULAR RECOGNITION IN CHEMISTRY, KLUWER, DORDRECHT, NL, Bd. 23, 1995, Seiten 119-126, XP009046559 ISSN: 0923-0750 in der Anmeldung erwähnt

## Beschreibung

Gegenstand der vorliegenden Erfindung sind neue Verwendungen von Liponsäure-haltigen Cyclodextrin-Komplexen, bestehend aus mindestens einem Vertreter der Reihe unsubstituiertes α-, β- und γ-Cyclodextrin sowie mindestens einem Vertreter der Reihe racemische α-Liponsäure und racemische Dihydro-Liponsäure im nicht-medizinischen und im medizinischen Bereich.

Bei α-Liponsäure (Thioctsäure) handelt sich um eine 1,2-Dithiacyclopentan-3-Vateriansäure, die sowohl in racemischer Form als auch in Form der (R)-bzw. (S)-Enantiomerform existiert. Die α-Liponsäure stellt einen wichtigen Bestandteil des Zellstoffwechsels dar und ist daher auch in zahlreichen Pflanzen und tierischen Organismen zu finden. Sie wirkt u.a. als eines der Co-Enzyme bei der oxidativen Decarboxylierung von Pyruvat und anderen α-Ketosäuren.

Seit längerer Zeit wird α-Liponsäure zur Vorbeugung und Therapie verschiedener Erkrankungen eingesetzt, wobei vor allen Dingen Lebererkrankungen und Leberschädigungen sowie diabetische und alkoholische Polyneuropathien und Veränderungen peripherer Nerven, die auf Stoffwechselerkrankungen zurückgehen, im Vordergrund stehen.

Unter Dihydro-Liponsäure wird chemisch Di-6,8-dimercapto-Octansäure verstanden, welche die reduzierte Form der α-Liponsäure darstellt.

Neben den reinen Säureformen der α-Liponsäure und Dihydro-Liponsäure sind auch zahlreiche Derivate, wie Ester und Salze, bekannt.

Bei den Cyclodextrinen handelt es sich um zyklische Oligosaccharide, die aus 6, 7 oder 8 α(1-4)-verknüpften Anhydroglucose-Einheiten aufgebaut sind, wobei am bekanntesten die durch eine enzymatische Stärkekonversion hergestellten α-, β- oder γ-Cyclodextrin-Varianten sind, die sich hauptsächlich im Durchmesser ihrer hydrophoben Kavitäten unterscheiden und die sich insbesondere zum Einschluss von Substanzen mit überwiegend lipophilem Charakter eignen.

Einschlussverbindungen, die als Trägermaterial auf Cyclodextrine zurückgreifen, sind aus dem Stand der Technik bestens bekannt.

So wird beispielsweise in der deutschen Offenlegungsschrift DE 102 53 042 A1 eine kosmetische Zubereitung beschrieben, die einen Komplex aus Cyclodextrin und Vitamin F enthält. Die Cyclodextrin-Komponente wird dabei aus der Reihe α-, β- und γ-Cyclodextrin ausgewählt.

Die deutsche Offenlegungsschrift DE 102 00 657 A1 beschreibt einen 2:1 Komplex bestehend aus α- oder γ-Cyclodextrin und α-Tocopherol. Dieser Komplex kann in kosmetischen Formulierungen eingesetzt werden und weist gegenüber den entsprechenden physikalischen Mischungen eine deutlich höhere Stabilität auf.

Gegenstand der beiden europäischen Patentanmeldungen EP 654 484 A2 und EP 1 063 241 A1 sind jeweils Arzneimittelzubereitungen, die neben Liponsäure oder Dihydro-Liponsäure Cyclodextrine in Form von Einschlussverbindungen enthalten. Die in diesen beiden Dokumenten beschriebenen Einschlussverbindungen werden ausschließlich zur Herstellung von Arzneimitteln eingesetzt und enthalten entweder Liponsäure oder Dihydro-Liponsäure und ein substituiertes Cyclodextrin oder aber Cyclodextrin oder Cyclodextrin-Derivate in Kombination mit den Enantiomeren von Liponsäure oder Dihydro-Liponsäure. Es wurde festgestellt, dass die entsprechenden Einschlussverbindungen sehr stabil sind. Bevorzugte Einsatzformen der beschriebenen Medikamente sind Granulate, Kau- oder Brausetabletten.

Hergestellt werden diese Medikamente, indem die Liponsäure-Komponente in Wasser suspendiert wird, dann die Cyclodextrin-Komponente zugegeben und das gesamte Reaktionssystem abgekühlt wird; abschließend isoliert man die erhaltene Einschlussverbindung.

Aus der internationalen Patentanmeldung WO 2000/64440 ist ein Verfahren zur Prophylaxe von Schädigungen bekannt, die aus Verletzungen des Rückenmarkes oder dessen Erkrankungen zurückgehen. Zum Zwecke der Vorbeugung wird eine bestimmte Menge von α-Liponsäure oder Dihydro-Liponsäure an Vertebraten verabreicht, was vorzugsweise oral oder transdermal erfolgt und wofür insbesondere ein Cyclodextrin-Einschlusskomplex verwendet wird. Anwendung findet die beanspruchte Verabreichung im medizinischen Sektor in Verbindung mit sogenannten Dekompressionserkrankungen (decompression sickness; DCS).

Die internationale Patentanmeldung WO 02/052955 beschreibt pharmazeutische und diätetische Zusammensetzungen sowie funktionelle Nahrungsmittel, beispielsweise als ein wasserdispergierbares Lipid-Saccharid-Pulver, in dem die lipophilen Wirkstoffe mit Cyclodextrin komplexiert sind. Als oxidationshemmendes Vitamin kann Liponsäure dabei ein Bestandteil der Lipidmischung sein.

Wie bereits angegeben, zeichnen sich die aus dem Stand der Technik bekannten Cyclodextrin-/Liponsäure-Komplexe durch ihre ausgezeichnete Stabilität aus.

In diesem Zusammenhang wurden von Tong Lin-Hui et al. (Journal of Inclusion Phenomena and Molecular Recognition in Chemistry; 23:119 bis 126, 1995) Einschlusskomplexe von α- und β-Cyclodextrin mit α-Liponsäure untersucht, wobei insbesondere Beobachtungen an DL-α-Liponsäure im Vordergrund standen. Die entsprechenden festen Komplexe wurden gemäß dieser Publikation erhalten, indem equimolare Mengen von Cyclodextrin und α-Liponsäure in wässriger Lösung vermischt wurden, wobei in einem ersten Schritt α-Liponsäure in kleinen Mengen Ethanol angelöst wurde und β-Cyclodextrin als gesättigte Lösung und α-Cyclodextrin als 2,4%ige Lösung eingesetzt wurden. Die so erhaltenen präzipitierten Feststoffe wurden durch Filtration abgetrennt. Da mit Stabilitätsmessungen bei diesen Komplexen offensichtlich eine erhöhte Oberflächenaktivität der Liponsäure im komplexierten Zustand festgestellt wurde, wurde der Rückschluss gezogen, dass mögliche lokale Irritationen, die durch Liponsäure-haltige Arzneimittel verursacht werden, durch Liponsäure-haltige Cyclodextrin-Komplexe verbessert werden können.

Aus dem bislang bekannten Stand der Technik sind somit Liponsäure- bzw. Dihydro-Liponsäure-haltige Cyclodextrin-Komplexe bekannt, die eine verbesserte Stabilität aufweisen. Diese Komplexe wurden bisher jedoch ausschließlich für den medizinischen Bereich bzw. zur Herstellung von Arzneimittel vorgesehen. Die in diesen Arzneimitteln enthaltenen Cyclodextrin-Komplexe zeichnen sich durch ein spezifisches Verhältnis zwischen der Cyclodextrin-Komponente und der α-Liponsäure-/Dihydro-Liponsäure-Komponente aus, wobei zusätzlich vorzugsweise substituierte Cyclodextrine eingesetzt werden.

Für die vorliegende Erfindung hat sich die Aufgabe gestellt, neue Verwendungsgebiete von Liponsäure-haltigen Cyclodextrin-Komplexen bereitzustellen.

Gelöst wurde diese Aufgabe durch die claim 2.

Bevorzugte nicht-medizinische Anwendungen sind die Stimulierung des Glukose-Metabolismus und/oder -Transports in Muskel- und/oder Fettzellen, die Stärkung des Energiestoffwechsels des Körpers und des Gehirns, die Steigerung der Ausdauer, Leistungsfähigkeit, Aufmerksamkeit, Konzentration und des Gedächtnisses sowie die Pflege zumindest teilweise verhornter Körperteile, insbesondere der Haut, Haare, Finger- und Fußnägel.

Weitere bevorzugte nicht-medizinische Anwendungen sind der Einsatz der Komplexe als Mittel für die klinische Ernährung im Rahmen der Prophylaxe oder Therapie von Erkrankungen, z.B. zur Vorbeugung und/oder Behandlung von Symptomen des inflammatorischen Formenkreises und insbesondere von Arthritis, von Beeinträchtigungen der Leberfunktion, und insbesondere von Alkoholintoxikationen, von Parästhesien und Neuropathien, von Mitteln mit cytoprotektiven und/oder antiphlogistischen und/oder antinociceptiven (analgetischen) Eigenschaften und/oder Eigenschaften, die der Bildung von Radikalen entgegenwirken, und zur Prävention und/oder Behandlung von Diabetes Typ 2. Im Rahmen der nicht-medizinischen Anwendungen können die Komplexe als nicht verschreibungspflichtige Mittel, z.B. in Form von Nahrungsergänzungsmitteln, Funktionsnahrungsmitteln und Mitteln für die klinische Ernährung gegebenenfalls gemeinsam mit verschreibungspflichtigen pharmazeutischen Mitteln verabreicht werden.

Ein weiterer Aspekt der Erfindung sind neue medizinische Anwendungen der Komplexe zur Vorbeugung und/oder Behandlung von Symptomen des inflammatorischen Formenkreises und insbesondere von Arthritis, von Beeinträchtigungen der Leberfunktion, und insbesondere von Alkoholintoxikationen, von Parästhesien und Neuropathien, von Mitteln mit cytoprotektiven und/oder antiphlogistischen und/oder antinociceptiven (analgetischen) Eigenschaften und/oder Eigenschaften, die der Bildung von Radikalen entgegenwirken, und zur Prävention und/oder Behandlung von Diabetes Typ 2.

Als besonders geeignet hat sich die beanspruchte Verwendung zur Herstellung eines Mittels herausgestellt, welches zur Beeinflussung des Blutglukosespiegels und/oder der Glukagon-ähnlichen Peptid-1(GLP-1)-Aktivität und/oder des Bindungsverhaltens zwischen GLP-1 und dem GLP-1-Rezeptor und/oder der Insulin-Resistenz und/oder der *in vivo*-Umsetzung von Glukose zu Glykogen und/oder der Expression des Insulin-Rezeptor-Substrat-2 (IRS-2)-Polypeptids und/oder der Insulin-stimulierten Glukoseaufnahme und/oder der hepatischen Glukosebildung geeignet ist.

Neben den eben geschilderten Einsatzgebieten kommt auch insbesondere die Verwendung zur Vorbeugung und Selbstmedikation von Formenkreisen in Frage, die im Zusammenhang mit Hyperlipidämie, Hypertension, Fettsucht und Übergewicht, Artheriosklerose und Diabetes stehen.

Neben der hohen Stabilität dieser Komplexe hat sich herausgestellt, dass zusätzlich der üblicherweise von der Liponsäure-Komponente ausgehende unangenehme Geruch deutlich verbessert oder gänzlich vermieden werden kann und dass außerdem das ebenfalls bekannte störende Kratzgefühl bei der Einnahme höherer Liponsäuremengen im Hals vollständig unterdrückt werden kann. Hinzu kommt die Beobachtung, dass bei der erfindungsgemäßen Verwendung von Liponsäure-haltigen Cyclodextrin-Komplexen diese in basischem Milieu, wie sie beispielsweise durch entsprechende Komponenten in Blends im nicht-medizinischen Bereich verursacht werden oder durch Temperatureinflüsse in Folge der nichtstandardisierten Lagerung, wie sie häufig beim Endverbraucher stattfindet, äußerst stabil gegenüber Polymerisationsvorgängen sind.

Insbesondere die Verbesserungen in Form eines verminderten Kratzempfindens bzw. die Unterdrückung des sonst störenden Geruches führen auch ohne eine zusätzliche Verkapselung des Komplexes oder anderweitiger Formulierungsmaßnahmen zu einem deutlich besseren Compliance-Verhalten, was vor allem für die beanspruchte Verwendung im nicht-medizinischen Bereich in Form von Nahrungsergänzungsmitteln, Funktionsnahrungsmitteln und auch im kosmetischen Bereich von großer Bedeutung ist und so nicht zu erwarten war.

Als besonders vorteilhaft hat es sich herausgestellt, wenn ein Komplex verwendet wird, der mindestens 20 Gew.-% der Cyclodextrin-Komponente enthält, wobei Anteile zwischen 30 und 99,9 Gew.-% als besonders bevorzugt anzusehen sind; in diesem Zusammenhang empfiehlt es sich besonders, die Cyclodextrin-Komponente in Lebensmittelqualität einzusetzen, wobei für die beanspruchte Verwendung insgesamt α-Cyclodextrin besonders zu empfehlen ist.

Da die erfindungsgemäße Verwendung nicht auf eine bestimmte Zusammensetzung beschränkt ist, können die Anteile der beiden Hauptkomponenten Cyclodextrin und α-Liponsäure bzw. Dihydro-Liponsäure in weiten Bereichen variiert werden; jedoch haben sich Mengenverhältnisse als empfehlenswert herausgestellt, bei denen die Komponenten Cyclodextrin und (Dihydro-)Liponsäure im Verhältnis von 5 bis 99:1 vorliegen. Im Rahmen der vorliegenden Erfindung werden Komplexe als bevorzugt betrachtet, die 20 bis 95 Gew.-%, insbesondere 60 bis 90 Gew.-% und vor allem 70 bis 85 Gew.-% der Cyclodextrin-Komponente und 80 bis 5 Gew.-%, insbesondere 20 bis 15 Gew.-% und insbesondere 15 bis 10 Gew.-% der Liponsäure-Komponente enthalten.

Da die beiden genannten Hauptkomponenten in ihrer Gesamtheit somit immer im Überschuss vorliegen, sind mögliche Verunreinigungen aufgrund des jeweils verwendeten Herstellungsverfahrens von nur untergeordneter Bedeutung. Dies ist auch der Grund dafür, dass von der vorliegenden Erfindung auch eine Variante berücksichtigt wird, bei der ein Komplex verwendet wird, der zusätzlich aus Wasser und Einsatz- bzw. Nebenreaktionsprodukten aus der Komplexherstellung besteht.

Die vorgeschlagene neue Verwendung wird folgendem Verfahren hergestellt:

Zunächst wird im Verfahrensschritt a) eine wässrige Lösung vorgelegt, die vorzugsweise basischen Charakter hat, was sich aufgrund der allgemein bekannten physikalisch-chemischen Eigenschaften der α-Liponsäure als vorteilhaft erwiesen hat; dann wird im Verfahrensschritt b) die jeweilige Liponsäure-Komponente hinzugefügt, anschließend im Schritt c) die Cyclodextrin-Komponente hinzugefügt, die vorzugsweise in Pulverform vorliegt, und anschließend d) wird die aus den Verfahrensschritten a) bis c) erhaltene Lösung bis maximal zu deren Klarpunkt gerührt. Anschließend wird im Schritt e) der Cyclodextrin-/Liponsäure-Komplex mit Hilfe einer mineralischen Säure ausgefällt, wofür sich Salzsäure besonders eignet. Abschließend wird im Verfahrensschritt f) das erhaltene Ausfällungsprodukt getrocknet. Zur Einstellung des basischen Charakters der im Schritt a) vorgelegten wässrigen Lösung sollte vorzugsweise eine Alkalilauge und insbesondere NaOH eingesetzt werden. Das Ausfällen im Schritt e) gelingt besonders gut unter Rühren.

Die jeweils erhaltenen Komplexe können direkt wie beansprucht verwendet werden, sie können aber auch durch zusätzliche Filtrationsschritte, eine Zentrifugation oder Trocknung, durch Vermahlen, Sieben, Sichten und Granulieren sowie Tablettieren entsprechend isoliert und vorher aufbereitet werden.

Wie bereits angedeutet, können die jeweils racemische α-Liponsäure und die Dihydro-Liponsäure auch in Form geeigneter Derivate im Sinne der vorliegenden Erfindung verwendet werden. Bevorzugte Derivate der α-Liponsäure und der Dihydro-Liponsäure sind Ester und Salze. Als Salzbildner gelten typischerweise basische Aminosäuren, wie Arginin oder Lysin, physiologisch verträgliche Alkali- oder Erdalkali-Hydroxybicarbonate oder -Hydrogencarbonate, Ammoniumhydroxyd, Amine der Formel N R¹, R², R³, worin die Reste R¹ bis R³ gleich oder verschieden sind, und Wasserstoff, ein C₁₋₄-Alkyl oder C₁₋₄-Hydroxyalkyl bedeuten, wie sie beispielsweise Mono-Diethanolamin, 1-Amino-2-Propanol und 3-Amino-1-Propanol darstellen; es kommen aber auch Alkylendiamine mit einer Alkylenkette aus 2 bis 6 Kohlenstoffatomen, wie Ethylendiamin oder Hexamethylentetramin, gesättigte cyclische Aminoverbindungen mit 4 bis 6 RingKohlenstoffatomen, wie beispielsweise Piperidin, Piperazin, Pyrrolidin und Morpholin, in Frage. Ebenfalls geeignete Salzbildner sind N-Methylglucamin, Kreatin, Tromethamol und N-Methylmorpholin.

Wie ebenfalls bereits beschrieben, können erfindungsgemäß nicht nur Komplexe verwendet werden, die ausschließlich Cyclodextrin und (Dihydro-) Liponsäure enthalten, sondern darüber hinaus auch noch herstellungsbedingt Wasser und andere Einsatz- bzw. Nebenreaktionsprodukte. Von der vorliegenden Erfindung wird aber auch eine Variante berücksichtigt, bei der die beanspruchte Verwendung der Herstellung eines Mittels dient, das neben dem Cyclodextrin-/Liponsäure-Komplex noch weitere bioaktive Komponenten, Aromen und/oder Texturierungsmittel enthält. Zu berücksichtigen sind hierbei insbesondere als physiologisch aktive Komponente Guanidin-Derivate, wie Kreatin, Kreatinol und Guanidinoessigsäure, aber auch Phospholipide, wie Phosphatidylcholin, Phosphatidylserin, Phosphatidylethanolamin, Phosphatidylinositol und Phosphatidsäure; des Weiteren kommen ungesättigte Fettsäuren, Phytosterole und natürliche Extrakte und hier insbesondere von Arthemisia, sowie Vitamine, insbesondere der Gruppen C und/oder E, Aminosäuren und natürlich deren Mischungen in Frage.

Natürlich können im Rahmen der vorliegenden Erfindung auch Komplexe verwendet werden, die neben den beschriebenen bioaktiven Komponenten noch zusätzlich Formulierungshilfsmittel enthalten, die insbesondere im nicht-medizinischen Bereich die Attraktivität und Compliance für den Endverbraucher erhöhen. Hier sind insbesondere Formulierungshilfsmittel vom Typ der Aromen und Farbstoffe, aber auch Geschmacksverbesserer und Geschmacksverstärker zu nennen. Wenn, wie ebenfalls beansprucht, die Verwendung zur Herstellung einer kosmetischen Zubereitung im Vordergrund steht, kommen als weitere Komponenten Silikonöle, Feuchthaltemittel, die die Haut oder die Haare vor dem Austrocknen schützen, so genannte Emolliants, also typische Pflegemittel, Gelbildner und Emulgatoren in Betracht. Möglich ist aber auch die Abmischung mit Sonnenschutzfiltern, Selbstbräunungsmitteln und Vitaminen und sonstigen geeigneten Komponenten, wie sie in kosmetischen Formulierungen in Form von Lotionen, Gels, Pudern, Masken, Cremes (z.B. Wasser in Öl oder Öl in Wasser Emulsionen), Packungen, Pflegestiften, Sprays und Aerosolen für die topische Anwendung geeignet sind.

Zusammenfassend kann festgestellt werden, dass mit der vorliegenden Erfindung im Prinzip die bekannten Cyclodextrin-(Dihydro-)-Liponsäure-Komplexe aufgrund deren spezifischer Komponenten einem neuen Verwendungszweck im nicht-medizinischen Anwendungsbereich zugeführt werden konnten. Die hierfür eingesetzten Komplexe zeichnen sich neben der bekannten Stabilität durch das Fehlen der sonst für die Liponsäure-Komponente bekannten negativen Merkmale, wie Kratzen im Hals und störende Gerüche, aus, wodurch die ebenfalls sonst üblichen zusätzlichen Formulierungsmaßnahmen, wie Verkapselung oder sonstige Formulierungsmaßnahmen, entfallen können. Auf äußerst wirtschaftliche Weise können somit die entsprechenden Komplexe dem Gebiet der Selbstmedikation zugeführt werden, welches bekanntermaßen durch ein besonders kritisches Verbraucherverhalten geprägt ist. Im Gegensatz zum medizinischen Anwendungsgebiet, bei dem die Selbstauswahl und Entscheidungsfindung durch den Endverbraucher weitgehend zurückgedrängt ist, können die beanspruchten Nahrungsergänzungsmittel, Funktionsnahrungsmittel, die Mittel zur klinischen Ernährung und kosmetische Zubereitungen so zur Verfügung gestellt werden, dass sie die Verbraucherwünsche erfüllen. Dabei kann die Darreichungsform in weiten Bereichen variiert werden, wobei neben den eben beschriebenen kosmetischen Formen bei oraler Verabreichung Pulver, Granulate, Dragees, Drops, Getränke, Säfte und Milchprodukte, verschiedene Schokoladenformen, Riegel, Kaugummis und Weichschaumzubereitungen in Frage kommen.

Die nachfolgenden Beispiele verdeutlichen die angesprochenen Vorteile der beanspruchten Verwendung.

### Beispiele

### 1. Herstellung von α-Liponsäure/Cyclodextrin-Komplexen

30,5 kg NaOH wurden unter Rühren in 1,0 l (1000 kg) Wasser eingetragen. Anschließend wurde diese basische wässrige Lösung mit 45,5 kg racemischer α-Liponsäure versetzt und die daraus erhaltene klare Lösung mit 180,9 kg der α-Cyclodextrin-Komponente (Cavamax^{R} W6 von Fa. Wacker Chemie GmbH). Nachdem diese Lösung bis zum Klarpunkt gerührt worden war, wurde als mineralische Säure 22,7 kg HCl unter rühren zugesetzt und auf diese Weise der gewünschte Komplex ausfällt. Der Komplexbildungsvorgang wurde abgeschlossen, indem die Lösung für weitere 40 Minuten bei 22 °C gerührt wurde. Abschließend wurde der Komplex durch Filtration abgetrennt und bei Temperaturen von 110 °C getrocknet.

### 2. Stabilitätsuntersuchungen

Zunächst wurde eine Blendformulierung mit folgender Zusammensetzung hergestellt:

50 g Kreatin-Monohydrat; 20 g Zitronensäure; 5,0 g Natriumkarbonat; 15 g Natriumbikarbonat; 5,0 g Zitronenaroma; 0,5 g Aspartam; 0,5 g Acesulfam K; 4,0 g Magnesiumhydroxid

Zu diesem Blend wurden 0,2 Gew.-% α-Liponsäure bzw. 1,67 Gew.-% eines racemische α-Liponsäure-/α -Cyclodextrin-Komplexes gegeben. Die jeweils erhaltenen Präparate wurden luft- und lichtgeschützt abgefüllt und bei 20 °C bzw. 40 °C gelagert.

Die nachfolgende Tabelle 1 verdeutlicht die Ergebnisse dieses Lagertests:

**Tabelle 1 (Stabilitätstest):**

| α-Liponsäure-Gehalt (Gew.-%) des Blends mit 0,2 % ALA bei 20 °C | | | | | |
|---|---|---|---|---|---|
| 1 Monat | 2 Monate | 3 Monate | 6 Monate | 9 Monate | 12 Monate |
| 0,19 | 0,17 | 0,16 | 0,14 | 0,12 | 0,1 |
| | | | | | |

| α-Liponsäure-Gehalt (Gew.-%) des Blends mit 0,2 % ALA bei 40 °C | | | | | |
|---|---|---|---|---|---|
| 1 Monat | 2 Monate | 3 Monate | 6 Monate | 9 Monate | 12 Monate |
| 0,16 | 0,11 | 0,09 | 0,06 | 0,03 | 0 |
| | | | | | |

| α-Liponsäure-Gehalt (Gew.-%) des Blends mit 1,67 % ALA-CD bei 20 °C | | | | | |
|---|---|---|---|---|---|
| 1 Monat | 2 Monate | 3 Monate | 6 Monate | 9 Monate | 12 Monate |
| 0,2 | 0,2 | 0,2 | 0,2 | 0,2 | 0,2 |
| | | | | | |

| α-Liponsäure-Gehalt (Gew.-%) des Blends mit 1,67 % ALA-CD bei 40 °C | | | | | |
|---|---|---|---|---|---|
| 1 Monat | 2 Monate | 3 Monate | 6 Monate | 9 Monate | 12 Monate |
| 0,2 | 0,2 | 0,2 | 0,2 | 0,2 | 0,2 |

### 3. Physiko-chemische Eigenschaften

Der unter 2 beschriebene Blend wurde ebenfalls mit 0,2 Gew.-% einer racemischen α-Liponsäure bzw. 1,67 Gew.-% eines racemischen α-Liponsäure/α-Cyclodextrin-Komplexes versetzt und mit Hilfe einer Tablettiermaschine zu Brausetabletten verpresst.

Die Brausetabletten wurden luft- und lichtgeschützt bei 20 °C bzw. 40 °C gelagert, wobei sich ein analoger Stabilitätsverlauf wie im Stabilitätsbeispiel 2 gezeigt hat.

Es konnte gezeigt werden, dass der Komplex durch mechanische Beanspruchungen nicht zerstört wird.

### 4. Bioverfügbarkeit

833 mg eines gemäß Beispiel 1 hergestellten racemische α-Liponsäure/α -Cyclodextrin-Komplexes (Charge B) und 100 mg einer racemischen α-Liponsäure (Charge A) wurden in Gelatinehartkapseln gefüllt. Sechs freiwillige Probanden (4 männlich; 2 weiblich; durchschnittliches Alter: 27,5 Jahre) erhielten jeweils 1 Kapsel der Charge A und der Charge B an jeweils drei aufeinander folgenden Wochen, morgens im Nüchtemzustand im Rahmen einer zweifachen Cross-Over-Studie. Die Blutentnahmen erfolgten zu sechs Zeitpunkten über eine Braunüle. Nach Gewinnung des Blutserums wurde dieses aliquotiert und in flüssigem Stickstoff gelagert. Die Analyse der Serumproben ergab folgendes Ergebnis:

Für die einzelnen Bioverfügbarkeitsläufe wurde die jeweilige AUC (Area under the serum concentration vs. time curve) errechnet und die Gesamt-AUC der Mittelwertsverläufe verglichen, wobei sich die Charge B durch eine verlängerte Absorption (Retardeffekt) auszeichnete.

Es wurden folgende AUC-Mittelwerte bestimmt:
Charge A: 4,246
Charge B: 2,775

Danach ergibt sich ein Verhältnis von Charge A zu Charge B von 1,5.

Es wurde festgestellt, dass die galenische Formulierung Charge B eine deutlich bessere Bioverfügbarkeit (1,5-fach) als die Formulierung Charge A aufweist. Insgesamt zeigt der α-Liponsäure/α-Cyclodextrin-Komplex (Charge B) einen deutlichen Vorteil gegenüber der nicht-komplexierten α-Liponsäure-Formulierung, was nicht nur auf quantitative Effekte zurückzuführen ist. Zusätzlich kann bei der Formulierung Charge B eine verlängerte Bioabsorption beobachtet werden.

## Patentansprüche

1. Verwendung von Liponsäure-haltigen Cyclodextrin-Komplexen, bestehend aus mindestens einem Vertreter der Reihe unsubstituiertes α-, β- und γ-Cyclodextrin sowie mindestens einem Vertreter der Reihe racemische α-Liponsäure, racemische Dihydro-Liponsäure und deren Derivaten, wobei der Komplex mit folgendem Verfahren hergestellt wird:
a) Vorlegen einer wässrigen, vorzugsweise basischen, Lösung, dann
b) Hinzufügen der Liponsäure-Komponente, anschließend
c) Hinzufügen der Cyclodextrin-Komponente, vorzugsweise in Pulverform, dann
d) Rühren der aus den Verfahrensschritten a) bis c) erhaltenen Lösung bis maximal zu deren Klarpunkt, anschließend
e) Ausfällen des Cyclodextrin/Liponsäure-Komplexes mit Hilfe einer mineralischen Säure, und abschließend
f) Trocknen des Ausfällungsproduktes,
als Nahrungsergänzungsmittel, Funktionsnahrungsmittel, Mittel für die klinische Ernährung und/oder als kosmetische Zubereitung im nicht-medizinischen Anwendungsbereich.

2. Verwendung nach Anspruch 1,
**dadurch gekennzeichnet,**
**dass** das Nahrungsergänzungsmittel, Funktionsnahrungsmittel, Mittel für die klinische Ernährung oder die kosmetische Zubereitung im nicht-medizinischen Anwendungsbereich zur Stärkung des Energiestoffwechsels des Körpers und des Gehirns, zur Steigerung der Ausdauer, Leistungsfähigkeit, Aufmerksamkeit, Konzentration und des Gedächtnisses oder zur Pflege zumindest teilweise verhornter Körperteile, insbesondere der Haut, Haare, Fingernägel und Fußnägel angewendet wird.

3. Verwendung nach Anspruch 1,
**dadurch gekennzeichnet,**
**dass** das Mittel für die klinische Ernährung im nicht-medizinischen Anwendungsbereich im Rahmen der Vorbeugung und/oder Behandlung von Symptomen des inflammatorischen Formenkreises und insbesondere von Arthritis, von Beeinträchtigungen der Leberfunktion, und insbesondere von Alkoholintoxikationen, von Parästhesien und Neuropathien, von Mitteln mit cytoprotektiven und/oder antiphlogistischen und/oder antinociceptiven (analgetischen) Eigenschaften und/oder Eigenschaften, die der Bildung von Radikalen entgegenwirken, von Mitteln zur Stimulierung des Glukose-Metabolismus und/oder -Transports in Muskel- und/oder Fettzellen oder von Mitteln zur Prävention und/oder Behandlung von Diabetes Typ 2 eingesetzt wird.

4. Verwendung nach Anspruch 3,
**dadurch gekennzeichnet,**
**dass** das Mittel zur Beeinflussung des Blutglukosespiegels und/oder der Glukagon-ähnlichen Peptid-1 (GLP-1)-Aktivität und/oder des Bindungsverhaltens zwischen GLP-1 und dem GLP-1-Rezeptor und/oder der Insulin-Resistenz und/oder der *in vivo*-Umsetzung von Glukose zu Glykogen und/oder der Expression des Insulin-Rezeptor-Substrat-2 (IRS-2)-Polypeptids und/oder der Insulin-stimulierten Glukoseaufnahme und/oder der hepatischen Glukosebildung geeignet ist.

5. Verwendung nach einem der Ansprüche 1 bis 4,
**dadurch gekennzeichnet,**
**dass** der Komplex mindestens 20 Gew.-% und bevorzugt 30 bis 99,9 Gew.-% der Cyclodextrin-Komponente, bevorzugt in Lebensmittelqualität und besonders bevorzugt als α-Cyclodextrin enthält.

6. Verwendung nach einem der Ansprüche 1 bis 5,
**dadurch gekennzeichnet,**
**dass** der Komplex aus 20 bis 95 Gew.-% der Cyclodextrin-Komponente und 80 bis 5 Gew.-% der Liponsäure-Komponente, insbesondere aus 60 bis 90 Gew.-% der Cyclodextrin-Komponente und 20 bis 15 Gew.-% der Liponsäure-Komponente und besonders bevorzugt aus 70 bis 85 Gew.-% der Cyclodextrin-Komponente und 15 bis 10 Gew.-% der Liponsäure-Komponente besteht.

7. Verwendung nach einem der Ansprüche 1 bis 6,
**dadurch gekennzeichnet,**
**dass** der Komplex zusätzlich aus Wasser und Einsatz- bzw. Nebenreaktionsprodukten aus der Komplexherstellung besteht.

8. Verwendung nach einem der Ansprüche 1 bis 7, wobei in Verfahrensschritt a) eine Alkalilauge und vorzugsweise KOH und im Verfahrensschritt e) Salzsäure, gegebenenfalls unter Rühren, eingesetzt werden.

9. Verwendung nach einem der Ansprüche 1 bis 8 zur Herstellung eines Mittels, das neben dem Cyclodextrin/Liponsäure-Komplex noch weitere bioaktive Komponenten, Aromen und/oder Texturierungsmittel enthält und insbesondere als physiologisch aktive Komponente Guanidin-Derivate wie Kreatin, Kreatinol und Guanidinoessigsäure, Phospholipide wie Phosphatidylcholin, Phosphatidylserin, Phosphatidylethanolamin, Phosphatidylinositol und Phosphatidsäure, ungesättigte Fettsäuren, Phytosterole und natürliche Extrakte, insbesondere von Arthemisia, Vitamine, insbesondere der Gruppe C und/oder E, Aminosäuren sowie deren Mischungen.

## Claims

1. Use of lipoic acid-containing cyclodextrin complexes consisting of at least one member of the series of unsubstituted α-, β- and γ-cyclodextrin, and also at least one member of the series of racemic α-lipoic acid, racemic dihydrolipoic acid and derivatives thereof, wherein the complex is produced by the following process:
a) initially charging an aqueous, preferably basic, solution, then
b) adding the lipoic acid component, subsequently
c) adding the cyclodextrin component, preferably in powder form, then
d) stirring the solution obtained from process steps a) to c) maximally to the clear point thereof, subsequently
e) precipitating out the cyclodextrin/lipoic acid complex using a mineral acid, and finally
f) drying the precipitation product,
as food supplements, functional foods, agents for clinical nutrition and/or as cosmetic preparation in the non-medical field of application.

2. Use according to Claim 1, **characterized in that** the food supplement, functional food, agent for clinical nutrition or the cosmetic preparation in the non-medical field of application is used for enhancing the energy metabolism of the body and of the brain, for increasing stamina, fitness,
attention, concentration and memory, or for the care of at least in part keratinous body parts, in particular the skin, hair, finger nails and toe nails.

3. Use according to Claim 1, **characterized in that** the agent for clinical nutrition in the non-medical field of application is used in the context of prevention and/or treatment of symptoms of the inflammatory disorder spectrum, and in particular arthritis, impairments of liver function, and in particular alcohol intoxications, of parasthesias and neuropathies, of agents having cytoprotective and/or antiphlogistic and/or anti-nociceptive (analgesic) properties and/or properties which counteract the formation of free radicals, of agents for stimulating the glucose metabolism and/or glucose transport in muscle cells and/or fat cells or of agents for the prevention and/or treatment of diabetes type 2.

4. Use according to Claim 3, **characterized in that** the agent is suitable for affecting the blood glucose level and/or glucagon-like peptide 1 (GLP-1) activity and/or the binding behavior between GLP-1 and the GLP-1 receptor and/or insulin resistance and/or the *in vivo* conversion of glucose to glycogen and/or expression of the insulin-receptor-substrate-2 (IRS-2) polypeptide and/or insulin-stimulated glucose uptake and/or hepatic glucose formation.

5. Use according to one of Claims 1 to 4, **characterized in that** the complex contains at least 20% by weight, and preferably 30 to 99.9% by weight, of the cyclodextrin component, preferably in food quality, and particularly preferably as α-cyclodextrin.

6. Use according to one of Claims 1 to 5, **characterized in that** 20 to 95% by weight of the complex consists of the cyclodextrin component and 80 to 5% by weight of the lipoic acid component, in particular 60 to 90% by weight of the cyclodextrin component and 20 to 15% by weight of the lipoic acid component, and particularly preferably 70 to 85% by weight of the cyclodextrin component, and 15 to 10% by weight of the lipoic acid component.

7. Use according to one of Claims 1 to 6, **characterized in that** the complex in addition consists of water and feed products or side reaction products of complex production.

8. Use according to one of Claims 1 to 7, wherein, in process step a), use is made of an alkali metal hydroxide solution, and preferably KOH, and in process step e) hydrochloric acid, if appropriate with stirring.

9. Use according to one of Claims 1 to 8 for producing an agent which, in addition to the cyclodextrin/lipoic acid complex, contains further bioactive components, flavorings and/or texturizing agents, and in particular as physiologically active component, guanidine derivatives such as creatine, creatinol and guanidinoacetic acid, phospholipids such as phosphatidylcholine, phosphatidylserine, phosphatidylethanolamine, phosphatidylinositol and phosphatidic acid, unsaturated fatty acids, phytosterols and natural extracts, in particular of arthemisia, vitamins, in particular of group C and/or E, amino acids and also mixtures thereof.

## Revendications

1. Utilisation de complexes de cyclodextrine contenant de l'acide lipoïque, consistant en au moins un représentant de la série de l'α-, β- et γ-cyclodextrine non substituée ainsi qu'en au moins un représentant de la série de l'acide α-lipoïque racémique, de l'acide dihydrolipoïque racémique et de leurs dérivés, où le complexe est produit avec le procédé suivant :
a) disposer au préalable une solution aqueuse, de préférence basique, puis
b) ajouter le composant acide lipoïque, ensuite
c) ajouter le composant cyclodextrine, de préférence sous forme de poudre, puis
d) agiter la solution obtenue à partir des étapes de procédé a) à c) au maximum jusqu'à son point de clarification, ensuite
e) précipiter le complexe cyclodextrine/acide lipoïque à l'aide d'un acide minéral, et enfin
f) sécher le produit précipité,
comme complément alimentaire, aliment fonctionnel, agent pour l'alimentation clinique et/ou comme préparation cosmétique dans le domaine d'utilisation non médical.

2. Utilisation selon la revendication 1 **caractérisée en ce que** le complément alimentaire, l'aliment fonctionnel, l'agent pour l'alimentation clinique ou la préparation cosmétique est utilisé dans le domaine d'utilisation non médical pour renforcer le métabolisme énergétique du corps et du cerveau, pour augmenter la durée, les performances, l'attention, la concentration et la mémoire ou pour les soins de parties du corps au moins partiellement calleuses, en particulier de la peau, des cheveux, des ongles des doigts et des ongles des pieds.

3. Utilisation selon la revendication 1 **caractérisée en ce que** l'agent pour l'alimentation clinique est utilisé dans le domaine d'utilisation non médical dans le cadre de la prévention et/ou du traitement des symptômes du domaine des formes inflammatoires et en particulier de l'arthrite, des atteintes de la fonction hépatique, et en particulier des intoxications alcooliques, des paresthésies et des neuropathies, d'agent ayant des propriétés cytoprotectrices et/ou antiphlogistiques et/ou antinociceptives (analgésiques) et/ou des propriétés qui s'opposent à la formation de radicaux, d'agent pour stimuler le métabolisme et/ou le transport du glucose dans les cellules musculaires et/ou adipeuses ou d'agent pour la prévention et/ou le traitement du diabète de type 2.

4. Utilisation selon la revendication 3 **caractérisée en ce que** l'agent convient pour influencer la concentration sanguine de glucose et/ou l'activité du peptide analogue au glucagon 1 (GLP-1) et/ou le comportement de liaison entre GLP-1 et le récepteur de GLP-1 et/ou la résistance à l'insuline et/ou la conversion *in vivo* du glucose en glycogène et/ou l'expression du polypeptide substrat de récepteur d'insuline 2 (IRS-2) et/ou l'absorption de glucose stimulée par l'insuline et/ou la formation de glucose hépatique.

5. Utilisation selon l'une des revendications 1 à 4 **caractérisée en ce que** le complexe contient au moins 20 % en poids et de préférence 30 à 99,9 % en poids de composant cyclodextrine, de préférence de qualité alimentaire et de manière particulièrement préférée sous forme d'α-cyclodextrine.

6. Utilisation selon l'une des revendications 1 à 5 **caractérisée en ce que** le complexe consiste en 20 à 95 % en poids de composant cyclodextrine et 80 à 5 % en poids de composant acide lipoïque, en particulier en 60 à 90 % en poids de composant cyclodextrine et 20 à 15 % en poids de composant acide lipoïque et de manière particulièrement préférée en 70 à 85 % en poids de composant cyclodextrine et 15 à 10 % en poids de composant acide lipoïque.

7. Utilisation selon l'une des revendications 1 à 6 **caractérisée en ce que** le complexe consiste en outre en eau et en produits utilisés ou de réaction secondaire provenant de la production du complexe.

8. Utilisation selon l'une des revendications 1 à 7 où, dans l'étape de procédé a), une lessive alcaline et de préférence KOH et dans l'étape de procédé e) l'acide chlorhydrique, sont utilisés, éventuellement sous agitation.

9. Utilisation selon l'une des revendications 1 à 8 pour la production d'un agent qui contient, outre le complexe cyclodextrine/acide lipoïque, encore d'autres composants bioactifs, des arômes et/ou des agents texturants et en particulier comme composant physiologiquement actif des dérivés de la guanidine comme la créatine, le créatinol et l'acide guanidinoacétique, des phospholipides comme la phosphatidylcholine, la phosphatidylsérine, la phosphatidyléthanolamine, le phosphatidylinositol et l'acide phosphatidique, des acides gras insaturés, des phytostérols et des extraits naturels, en particulier d'Artemisia, des vitamines, en particulier du groupe C et/ou E, des aminoacides ainsi que leurs mélanges.
